# EUROPEAN PATENT SPECIFICATION

(11) **EP 1 596 761 B1**
(45) Date of publication and mention of the grant of the patent: **17.06.2015**
(21) Application number: 04711431.9
(22) Date of filing: 16.02.2004
(51) Int. Cl.: A61F 2/06

(54) **STENT DELIVERY AND DEPLOYMENT SYSTEM**
STENTFREIGABE- UND ABLAGESYSTEM
SYSTEME D'AMENEE ET DE DEPLOIEMENT DE STENT

(30) Priority: 14.02.2003 US 447314 P
(43) Date of publication of application: 23.11.2005
(73) Proprietor: Salviac Limited, Dublin 2 (IE)
(72) Inventor: HORAN, Steven, Co. Westmeath (IE); VALE, David, Dublin 3 (IE); MOLLOY, Shane, Co. Galway (IE); BRADY, Earmon, Co. Roscommom (IE); KEEGAN, Martin, Co. Galway (IE)
(74) Representative: O'Brien, John Augustine
(86) International application number: PCT/IE2004/000023
(87) International publication number: WO 2004/071352

(56) References cited:
- EP-A- 1 095 634
- WO-A-01/12256
- WO-A-03/002019
- WO-A-03/002020
- US-A- 5 733 267
- US-A- 5 944 727
- US-B1- 6 464 721

## Description

### Introduction

The invention relates to a delivery system for delivery and deployment of a stent to a desired vascular location.

Vascular intervention is today undertaken to treat a large number of diseases that had heretofore been treated by surgery. Stents are used widely in a number of applications to provide structural support to vessels that are being treated.

Stents are commonly used in the repair of aneurysms, as liners for vessels, or to provide mechanical support to prevent the collapse of stenosed or occluded vessels. Stents are typically delivered in a compressed state to a specific location inside the lumen of a vessel or other tubular structure, and then deployed at that location in the lumen to an expanded state. A stent has a diameter in its expanded state which is several times larger that the diameter of the stent in its compressed state. Stents are also frequently deployed in the treatment of atherosclerotic stenosis in blood vessels, especially after percutaneous transluminal coronary angioplasty (PICA) procedures, to improve the results of the procedure and to reduce the likelihood of restenosis.

Stent designs are broadly divided into two categories, balloon expandable stents and self-expanding stents. The invention relates particularly to the delivery and positioning of self-expanding stents. The term self-expanding refers to the inherent material properties of the stent which cause the expansion of the stent once an external constraint has been removed. The effect is most commonly achieved by using a shape memory metallic alloy such as nitinol.

Generally, stents are delivered to the desired location by means of a catheter, specifically referred to as a delivery catheter. Delivery catheters are threaded through a guiding catheter to the site of the disease and once the correct position has been established by means of fluoroscopic or other imaging method, the stent is deployed.

Delivery systems for self expanding stents generally comprise an inner component or core about which the stent is positioned in a retracted or reduced diameter and an outer sheath surrounding the stent. The stent is deployed by retracting the outer sheath relative to the inner component. This has the effect of removing the constraint on the stent which, on release, expands into an increased diameter deployed configuration. The procedure is controlled by a clinician by manipulating various components outside of the vasculature.

Systems of this type are known from EP1095634, US5733267 and WO03/002020.

Conventional self expanding stent delivery systems suffer from the considerable disadvantage that they are difficult to use, even for a very skilled clinician. The clinician must hold some components steady whilst at the same time manipulating others. Even small irregular movements by the clinician may result in inaccurate deployment at the target site. Inaccurate deployment of a stent may diminish the effectiveness of supporting the vascular wall at the site of deployment.

There is therefore a need for an improves self expanding sent delivery system which will address these problems.

There are particular difficulties in navigating complex stent delivery systems through tortuous arterial passageways, especially a carotid artery, a superficial femoral artery or a renal artery.

One objective of the invention is to provide a stent delivery system that is capable of navigating significant tortuosity en route to a target location which can be a significant distance from the catheter entry location.

### Statements of Invention

According to the invention there is provided a delivery system for delivery and deployment of a self expanding stent to a desired vascular location of a patient, the system comprising;
a catheter shaft having a proximal end and a distal end, the distal end of the shaft defining a reception space for receiving a self expanding stent, the stent having a reduced diameter delivery configuration;
an inner core engagable with the proximal end of the stent;
an operator handle for movement of the catheter shaft relative to the inner core to deploy the self expanding stent;
a stabiliser component;
the inner core being fixed to the stabiliser component, at least during deployment of the self expanding stent.

In one embodiment the inner core has an abutment which is engagable with the proximal end of the stent to deploy the stent. The inner core may have a reduced diameter distal portion extending distally of the abutment at least partially through the stent in the reduced diameter delivery configuration of the stent. The inner core may form a tubular member in the region of abutment. The inner core may have a high compressive stiffness. The inner core may of a composite, or a metallic construction.

In one embodiment the catheter shaft comprises a distal sheath and a stent is frictionally coupled to the distal sheath in the delivery configuration. The inner core may have an abutment which is engagable with the proximal end of the stent to decouple the stent and distal sheath to deploy the stent.

The catheter shaft comprises a distal sheath portion and a proximal shaft portion, the diameter of the proximal shaft portion being smaller than the diameter of the distal sheath portion. The stabiliser component may be disposed over the smaller diameter proximal shaft. The stabiliser may comprise a tube and the diameter of the stabiliser tube is not greater than the diameter of the distal sheath of the catheter shaft.

The catheter shaft may have a guidewire exit port which is located proximally of the distal end of the catheter shaft. The guidewire exit port may be located proximally of the stent and/or proximally of the delivery sheath.

In one case the guidewire exit port is located at a transition between the distal sheath and the reduced diameter proximal shaft portion. The guidewire exit port may be located distally of the stabiliser component.

In one embodiment the guidewire exit port is configured to exit along an axis that is substantially parallel to a longitudinal axis of the distal sheath.

In another embodiment the system comprises a guidewire and the sum of the diameter of the guidewire and the diameter of the proximal shaft is less than the diameter of the distal sheath.

In one case the sum of the diameter of the guidewire and the diameter of the stabiliser component is less than the diameter of the distal sheath.

In one embodiment the inner core comprises a large diameter distal segment , a reduced diameter proximal segment, and a transition segment between the distal and proximal segments. The transition segment may be proximal of the abutment region. The transition segment may be distal of the exit port.

In one embodiment the stent directly engages the distal sheath and is slidable relative to the sheath. The distal sheath may be a composite with a low friction inner surface. In one case the distal sheath is reinforced to withstand the radial stresses of the stent in its constrained reduced diameter configuration.

In one embodiment the inner core is fixed to a component of the delivery system.

The component of the system to which the inner core is fixed may comprise the handle.

In one case the stabiliser component is fixed to a procedural catheter. In this case a haemostasis gasket may be provided between the stabiliser component and the procedural catheter.

Alternatively the catheter is an introducer sheath. The introducer sheath may have an integral haemostasis gasket.

Alternatively the procedural catheter is a guide catheter which may have a haemostasis gasket attachment. The gasket may be adjustable by the operator. The gasket attachment may be a Touhy Borst.

In one embodiment the system comprises a procedural guidewire and the guidewire is fixed or fixable to the stabiliser component.

In another embodiment the stabiliser component is length adjustable. The stabiliser component may comprise at least two parts which are movable relative to one another.

The stabiliser component position may be adjustable. For example, the stabiliser component may be adjusted by rotation of a threaded element which provides a position control device.

In one embodiment an intermediate component is provided between the stabiliser component and the inner core. The intermediate component may comprise the handle. The intermediate component may comprise at least one bridging piece. The bridging piece may extend through the wall of the proximal shaft. The bridging piece may project laterally of the inner core and/or the stabiliser component. In one case the bridging piece projects radially between the inner core and the stabiliser component.

In another embodiment the stabiliser component and the inner core are directly mounted to one another. The stabiliser component may be melded to the inner core, for example, by a welding, gluing, joining, laminating, or bonding process.

In another embodiement the stabiliser component and the inner core are directly mounted to one another proximal of the distal outer sheath. The stabiliser component and the inner core may be directly mounted to one another proximal of the outer shaft.

In a further embodiment the system includes a guidewire and the guidewire extends at least the length of the catheter shaft. In one case, the inner core defines a guidewire lumen along the length thereof.

The system may include a lock for the guidewire. The lock may be located proximal of the handle.

In another embodiment the stabiliser component comprises a tubular element and the tubular element has a tapered distal end.

In a further embodiment the system includes a guidewire and the guidewire is located within the profile of the stabiliser component

The stabiliser component may have a proximal opening to allow backflow of blood.

In another embodiment the stabiliser component extends substantially the length of the catheter shaft.

In another aspect the system provides a delivery system for delivery and deployment of a self expanding stent to a desired vascular location of a patient, the system comprising:
a catheter shaft having a proximal end and a distal end, the distal end of the shaft defining a reception space for receiving a self expanding stent, the stent having a reduced diameter delivery configuration;
an inner core engagable with the proximal end of the stent;
an external mounting for the inner core; and
an operator actuating element for the catheter shaft;
the operator actuating element being movable proximally of the external mounting for movement of the catheter shaft relative to the inner core to deploy the self expanding stent.

In one embodiment the operator handle is a pull handle for pulling the catheter shaft proximally relative to the inner core to deploy the self expanding stent. The catheter shaft and the operating handle may be interconnected by a connector. The connector may extend proximally of the external mounting. The connector may extend through the external mounting. The connector may comprise an elongate member such as a pull wire.

In one embodiment the inner core is fixed internal of the external mounting. A guidewire exit port may be provided at the proximal end of the external mounting.

The invention can be used for delivery and deployment of a self expanding stent comprising the steps of:
providing a delivery system comprising a catheter shaft having a proximal end and a distal end, the distal end of the shaft defining an outer sheath having a reception space for receiving a self expanding stent, the stent having a reduced diameter delivery configuration;
an inner core engaging the proximal end of the stent;
an operator handle for movement, of the catheter shaft relative to the inner core to deploy the self expanding stent;
introducing the delivery system into a vasculature of a patient;
delivering the stent delivery catheter to a region of interest;
fixing the inner core relative to the stabiliser component; and
deploying the self expanding stent by engaging the inner core with the proximal end of the stent.

The stent can be deployed by sliding the outer sheath and stent proximally to engage the inner core with the proximal end of the stent, the inner core engagement frictionally decoupling the stent and the sheath to deploy the stent.

In one case the stent is frictionally coupled to the outer sheath in the delivery configuration.

The method may comprise:
introducing a procedural guidewire into the vasculature;
advancing the guidewire to a region of interest; and
advancing the delivery system over the procedural guidewire.

In one example the method is of the rapid exchange type.

In one case the method comprises the steps of:
providing an embolic protection filter; and
deploying the filter distal of the region of interest, in advance of introduction of the delivery system.

The filter may be mounted on the guidewire or mountable to the guidewire.

In one embodiment the region of interest is a region of stenosis in an arterial vessel having a tortuous passageway leading thereto. The arterial vessel may typically be a carotid artery, a superficial femoral artery, or a renal artery.

In one embodiment the inner core is fixed relative to a component of the system. The component may be a guide catheter. The component may be a Touhy Borst.

In one embodiment the system comprises a stabiliser fixed at a proximal end to the handle and the method comprises fixing the stabiliser to a component of the system. In this case, the method may comprise fixing the distal end of the stabiliser to a guide catheter. The method may comprise fixing the distal end of the stabiliser to a Touhy Borst.

In a further aspect a method for delivery and deployment of a self expanding stent is described, comprising the steps of:
providing a delivery system providing:
   a catheter shaft having a proximal end and a distal end, the distal end of the shaft defining a reception space for receiving a self expanding stent, the stent having a reduced diameter delivery configuration;
   an inner core engagable with the proximal end of the stent;
   an external mounting for the inner core; and
   an operator actuating element for the catheter shaft; and
moving the operating actuating element proximally of the external mounting to move the catheter shaft relative to the inner core to deploy the stent.

In one embodiment the operator handle is a pull handle and the catheter shaft is pulled proximally of the inner core to deploy the stent.

### Brief Description of the Drawings

The invention will be more clearly understood from the following description thereof given by way of example only in which:-
Fig.1 is a side view of a stent delivery system of the invention;
Fig. 2 is an enlarged view of one region of Fig. 1;
Fig. 3 is an enlarged view of another region of Fig. 1;
Fig. 4 is a diagrammatic view of the delivery system of the invention in position in a tortuous anatomy of a patient;
Fig. 5 is an enlarged cross sectional view of one region of Fig. 4;
Fig. 6 is an enlarged cross sectional view of another region of Fig. 4;
Figs. 7(a) to 7(e) are diagrammatic side, partially cross sectional views illustrating various steps in using the stent delivery system of the invention;
Figs. 8(a) to 8(g) are diagrammatic side, partially cross sectional view illustrating various steps in a method of the invention;
Figs. 9(a) is a side view of the delivery system with a stent at a desired location constrained in a sheath;
Fig. 9(b) is a side view similar to Fig. 9(a) with the stent deployed;
Fig. 10 is a diagrammatic view of the delivery system in position in a patient;
Fig. 11 is an enlarged view of an indicated distal end region of the delivery system of Fig. 10;
Fig. 12 is an enlarged view of an indicated proximal end region of the delivery system of Fig. 10;
Figs. 13(a) to 13(c) are side cross sectional views of a proximal end of the delivery system in different positions of use;
Fig. 14 is a side view of an over the wire stent delivery system of the invention;
Fig. 15 is a side view of another over the wire stent delivery system of the invention;
Fig. 16 is a side cross sectional view of a proximal end of the delivery system of Fig. 15;
Fig. 17 is a view similar to Fig. 16 of a rapid exchange version of the delivery system;
Fig. 18 is a side, partially cross sectional view of an alternative delivery system in which a stabiliser comprises telescoping parts;
Fig. 19 is a side, partially cross sectional view of another delivery system in which a stabiliser is provided with a port;
Fig. 20 is a perspective view of a delivery system in which an outer sheath is accessed through a slot in the stabiliser;
Fig. 21 is a side, partially cross sectional view of a delivery system in which an outer sheath is accessed through a port at a proximal end of the system;
Fig. 22 is a side, partially cross sectional view of a delivery system in which a stabilising tube has a relatively small diameter;
Fig. 23 is a transverse cross sectional view at a proximal end of a delivery system in which the guidewire is not clamped on tightening of a Touhy Borst fitting;
Fig. 24 is a side partially cross sectional view of another delivery system with a stabiliser tube having a tapered distal end;
Fig. 25 is a perspective view of another delivery system of the invention;
Fig. 26 is a side, partially cross sectional view of a distal end of the delivery system of Fig. 25, in use;
Figs. 27 and 28 are side views of an alternative delivery system including a position control device; and
Fig. 29 is a cross sectional view of portion of another delivery system of the invention.

### Detailed Description

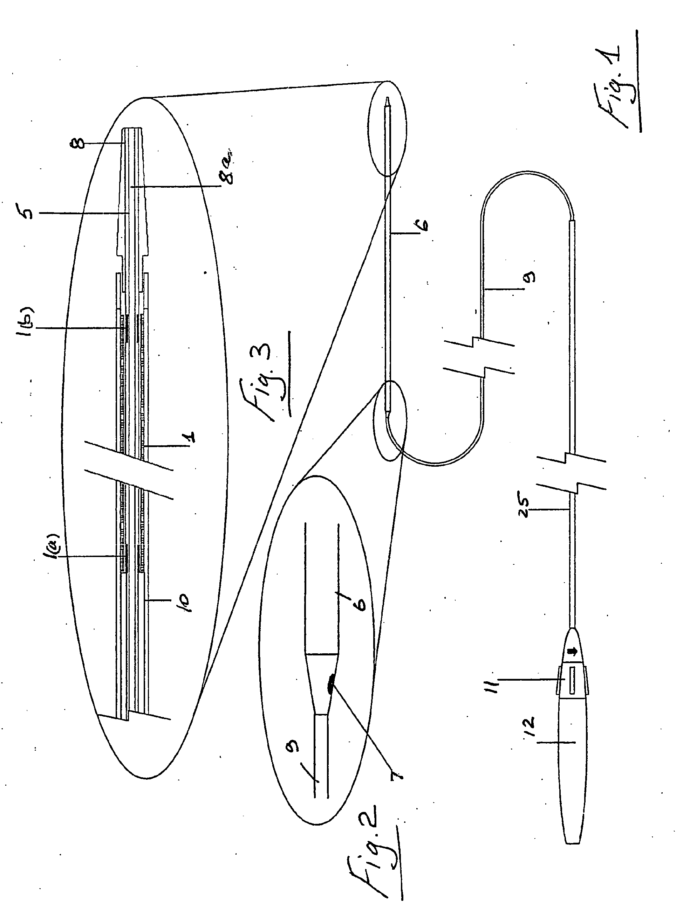

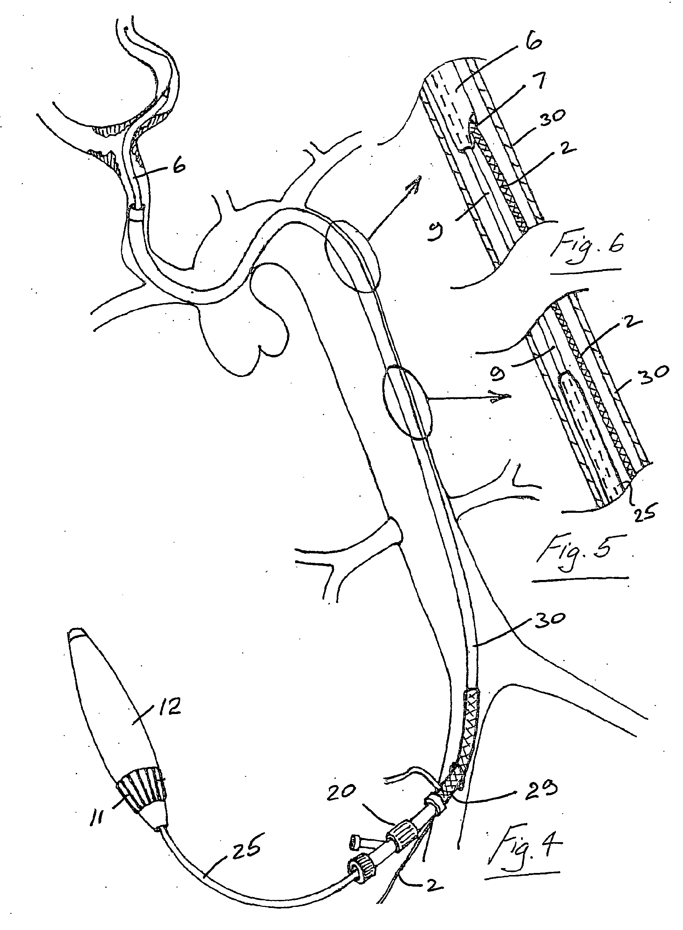

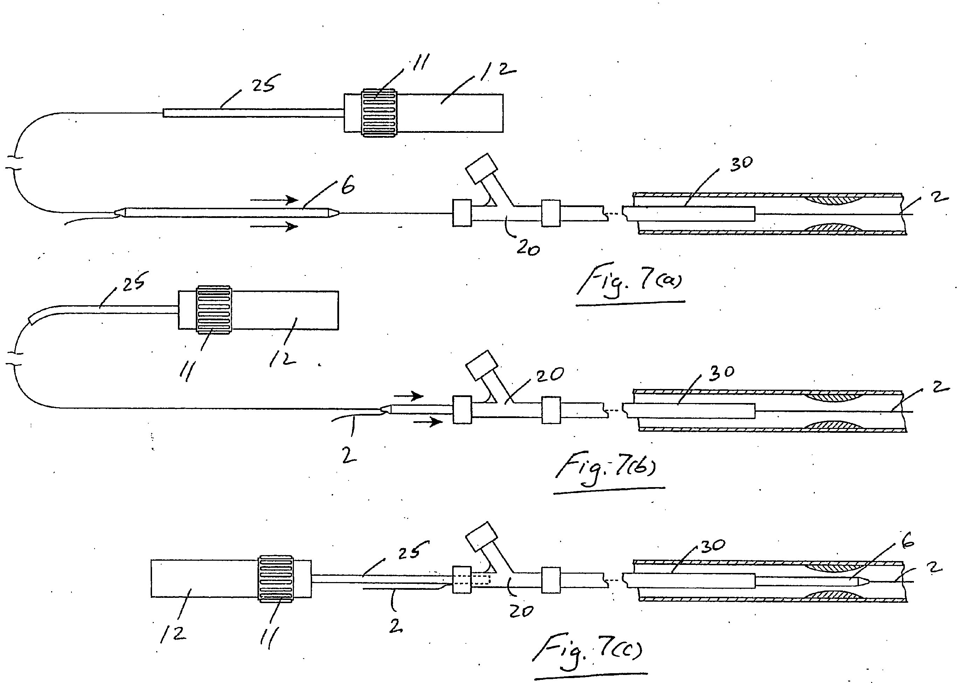

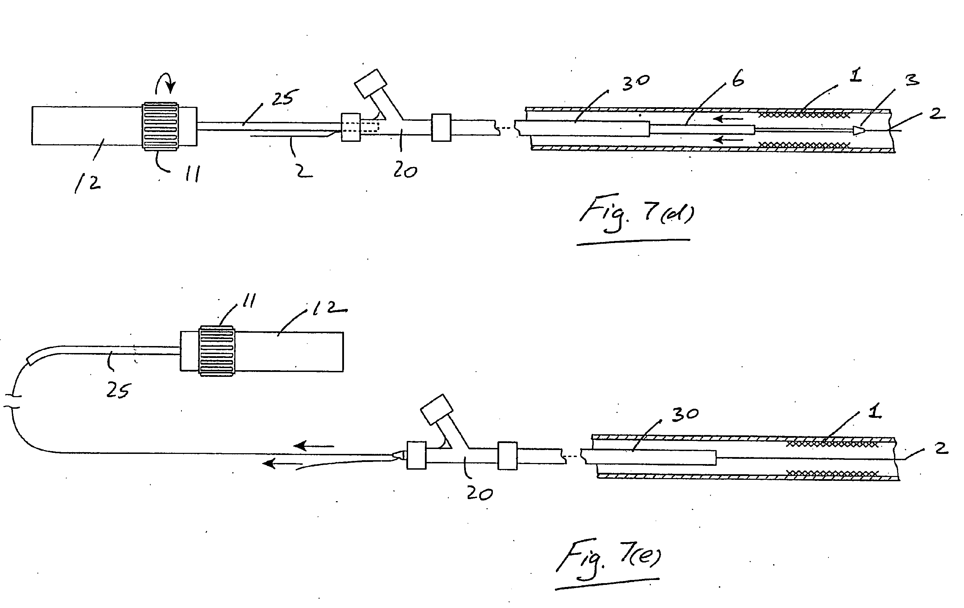

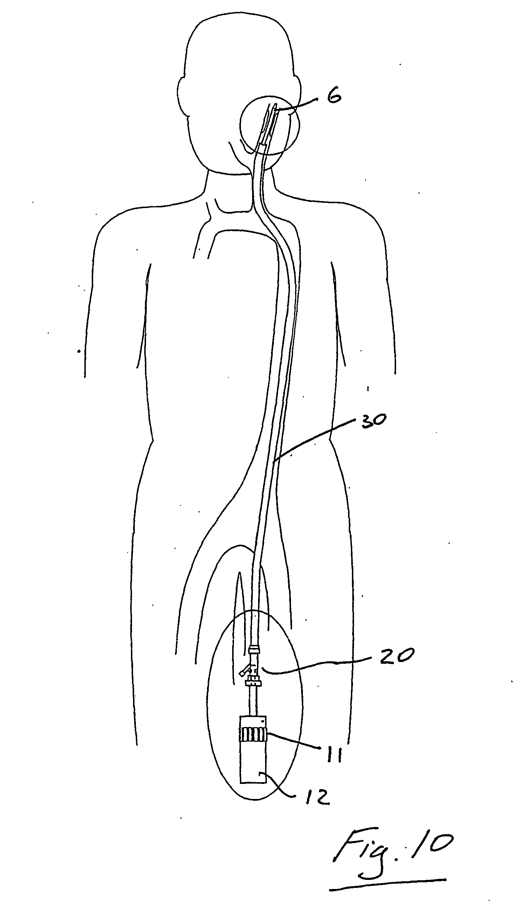

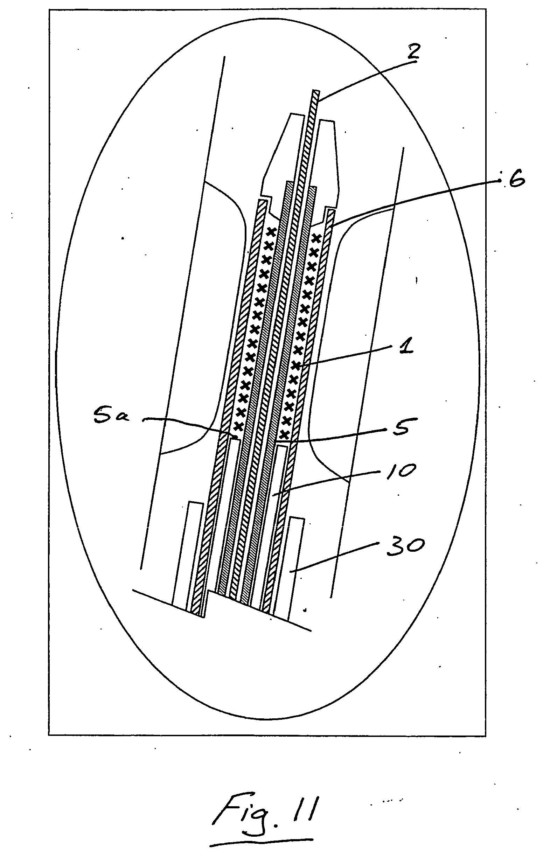

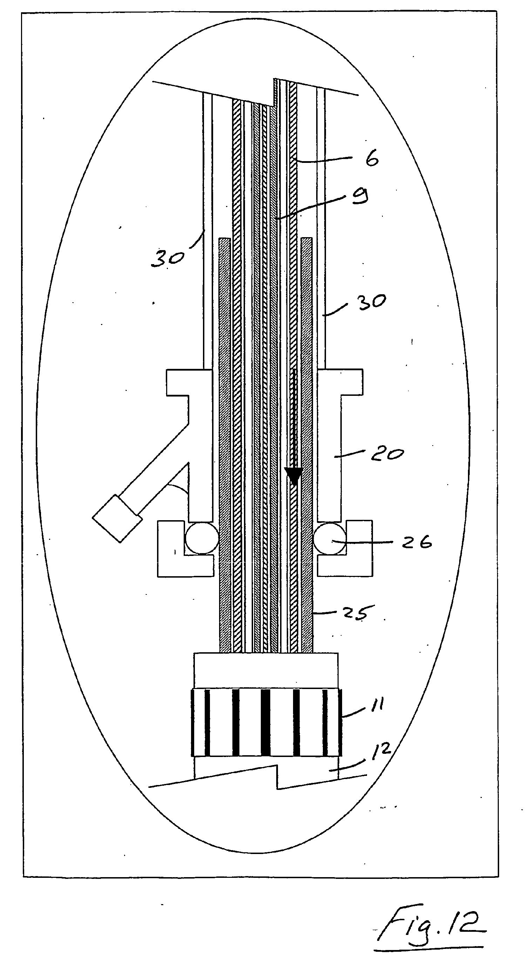

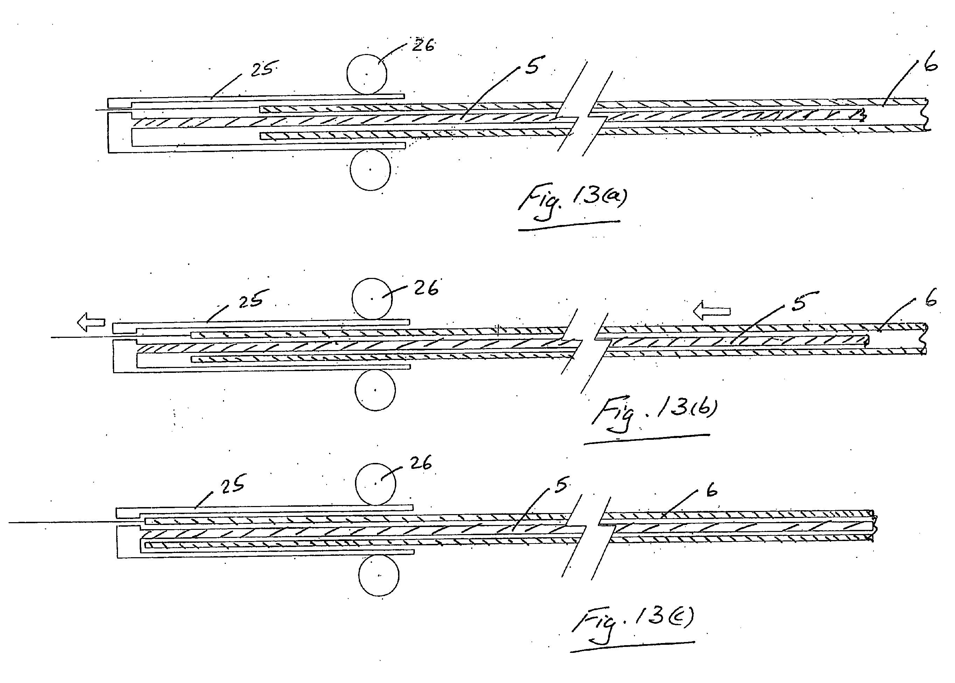

Referring to the drawings and initially to Figs. 1 to 13 thereof there illustrated a delivery and deployment system for a self expanding stent 1. The system is in this case configured for use with a guidewire 2 of the rapid exchange type with a distal tip 3.

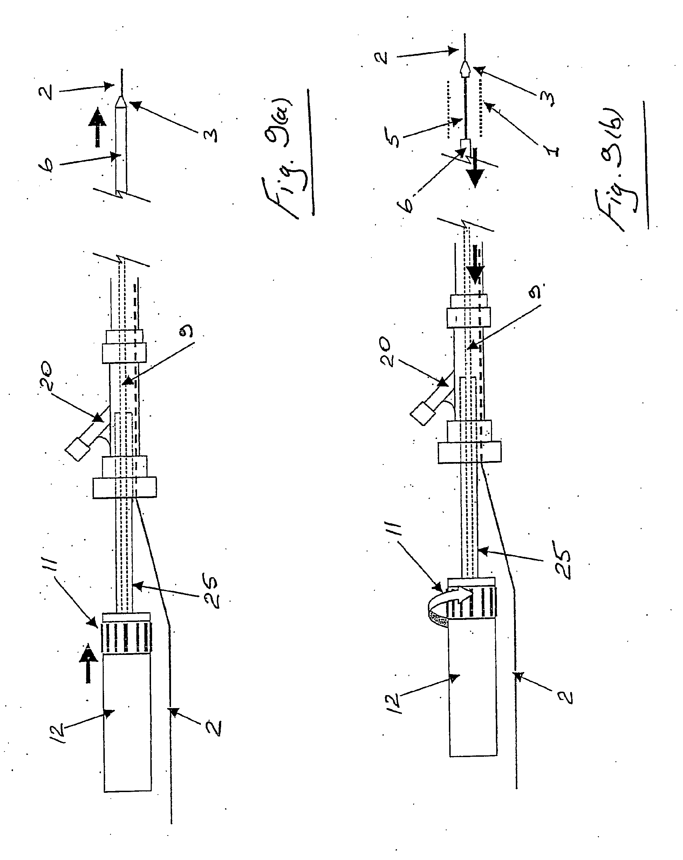

The system comprises an inner core 5 about which the stent 1 is located and a catheter shaft 9 having a distal sheath 6 which retains the stent 1 in a compressed configuration during delivery through the vasculature of a patient to a deployment site as illustrated for example in Fig. 9(a). To deploy the stent 1 the sheath 6 is drawn proximally relative to the inner core 5 and the stent expands into a deployed configuration as illustrated, for example in Fig. 9(b). The sheath 6 has a rapid exchange guidewire exit port 7.

The delivery system has a distal tip 8 with a guidewire lumen 8a. Marker bands 1a, 1b are provided for visualisation of the inner core 5 at the proximal and distal ends of the stent 1.

The inner core 5 is fixed to a larger diameter outer core 10, the difference in diameter providing a step or abutment 5a for engagement with the stent 1 for deployment. The inner and outer core 5, 10 are fixed to a handle 12 at the proximal end. The outer distal sheath 6 is connected to a catheter shaft 9 at the distal end and the catheter shaft 9 is connected at the proximal end to a deployment/actuating mechanism which in this case is operated by an actuator in this case in the form of a thumbscrew 11, rotation of the screw 11 being converted into linear movement of the sheath 6.

The stent 1 is frictionally coupled to the inside wall of the distal sheath 6 in the delivery configuration. In use, the inner core 5 abutment is engagable with the proximal end of the stent to decouple the stent 1 and the sheath 6 to enable the stent to be deployed. This, on sliding of the outer sheath 6 and stent 1 proximally the abutment of the inner core 5 is engaged with the stent 1 to frictionally decouple the stent 1 and sheath 6 to deploy the stent 1.

The catheter shaft 9 and the guidewire 2 in this case extend through a standard Touhy Borst fitting 20.

In the invention, the inner core 5 is locked relative to any suitable fixed location of the delivery system. In this case the delivery system comprises a stabiliser in the form of a stabiliser tube 25. The stabiliser 25 is clamped with an O-ring / seal 26 to the Tuohy Borst 20 which in turn is fixed to the handle 12. The stabiliser tube 25 is thereby fixed relative to the inner core 5 which controls the position of the stent 1 as it is deployed.

The stabilising tube 25 has sufficient hoop stiffness in the region at which it is clamped down by the Touhy Borst 20 so that it does not interfere with the outer sheath 6 or catheter shaft 9 as the stent 1 is deployed. In addition, the stabilising tube 25 has a bending stiffness so that it is flexible enough so that the handle 12 may be used at an angle to the Touhy Borst 20 but stiff enough so that the stent may be deployed even if the tube is in a bent configuration. The internal surface of the stabiliser tubing 25 has a low frictional surface to allow the outer sheath 6 to move relative to the stabilising tube 25 without affecting the deployment force of the stent 1. The external surface of the stabiliser tubing 25 may have sufficiently low friction so that the stent 1 may be repositioned without opening the Touhy Borst 20. However the tubing would not have so low a friction that the repositioning could happen unintentionally. The stabiliser tube 25 is of a low wall thickness, typically less than 0.203 mm (0.008 inches), preferably less than 0.152 mm (0.006 inches), and especially less than 0.076 mm (0.003 inches). It may be manufactured from any suitable high performance material such as PEEK (Polyether ketane); polyamide, Nylons such as Nylon 6, Nylon 66, Nylon 610, polymide, PEK (Polyether ketane); reinforced polymeric system such as braided systems, covered spring. In rapid exchange systems the stabiliser may be of PEEK or the like. In over the wire systems the stabliliser maybe of polyamide or the like.

The invention provides a delivery system which is stabilised to facilitate accurate deployment of a self expanding stent 1 at a desired location. The stabilisation is in this case achieved by fixing the inner core 5 to the Touhy Borst 20. The Touhy Borst 20 is connected to a guide catheter 30 which in turn is fixed to the patient's body. In the arrangement of Figs. 1 to 6 the stabiliser 25 comprises a tube that is connected to the inner core 5 at the proximal end and extends over the outer sheath 9. This allows the tube 25 to be fixed relative to the guide catheter 30 by tightening the Touhy Borst 20. As the outer sheath 9 is retracted the inner core 5 remains fixed relative to the patient. The inner core 5 holds the stent 1 in the same position throughout the deployment and thus an accurate deployment is achieved.

In use, an artery is accessed using a standard Seldenger technique. A short introducer sheath 29 is inserted at the access point to protect the artery at the proximal end. A dilator is then used to open the entry and a guidewire is introduced. A guide catheter 30 is then introduced over the guidewire and a Touhy Borst 20 is attached to the proximal end of the guide catheter 30. A procedural guidewire which is typically a 14 thou wire is introduced in a rapid exchange mode with a typical length of 190cm or, in on over the wire mode with a length of 320cm. An embolic protection filter may be introduced over the procedural guidewire and deployed distal of a site of interest. In the case of a lesion, the site may be pre-dilated using a balloon technique. The stent delivery catheter of the invention is then introduced and tracked over the guidewire to the location of interest. The sheathed stent is positioned relative to the target site. The Touhy Borst 20 is then locked to the stabiliser tube 25. With the stent 1 locked to the guide catheter 30 stent deployment is initiated, for example by moving the sheath 6 proximally relative to the inner core 5. The stent 1 is accurately deployed and the delivery system is then removed leaving the stent 1 in place.

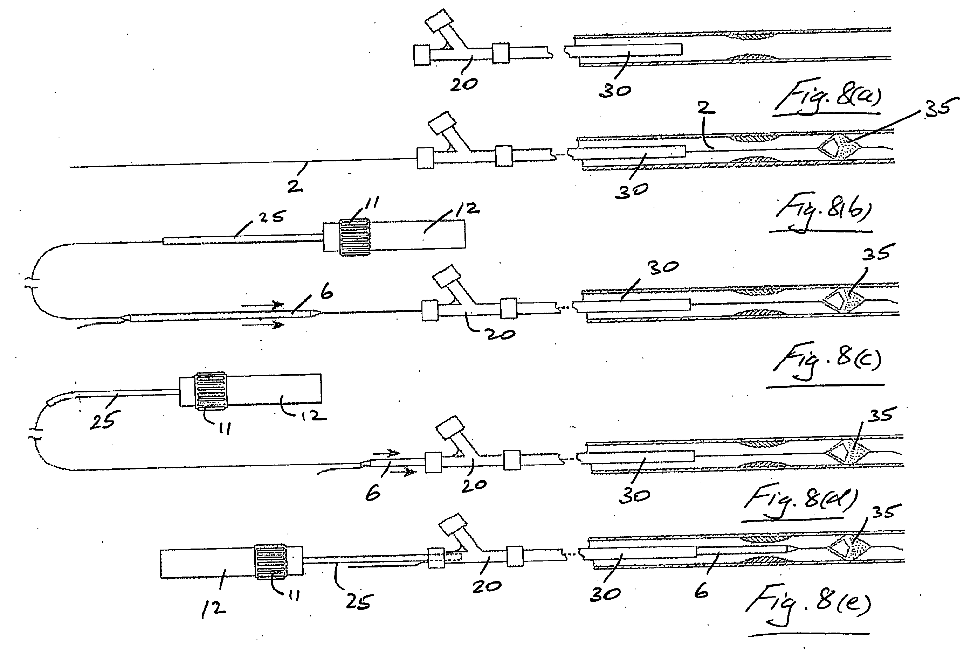

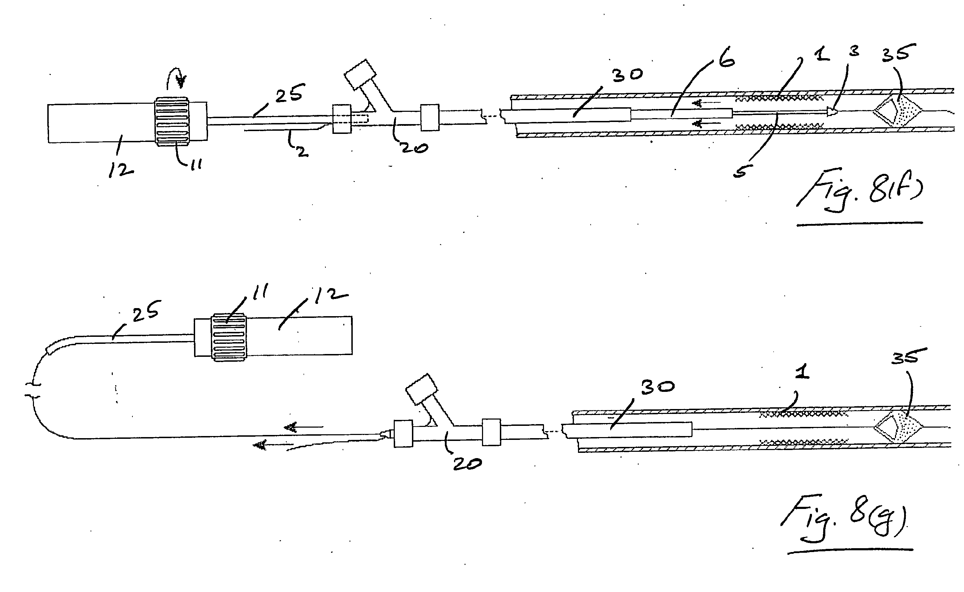

The delivery system may be used with an embolic protection filter 35 of the type described in WO 99/23976. The method involved is illustrated in Figs. 8(a) to 8(g). In this case a filter 35 is deployed distal of the site of interest (such a region of stenosis). The filter 35 may be mounted on a guidewire or may be delivered over a pre-advanced guidewire. The filter 35 is delivered in a collapsed form using a delivery catheter. The filter 35, on exiting the delivery catheter 36 self-expands or is expandable into the expanded deployed configuration located distal of the site of interest. Any embolic material which is dislodged during subsequent procedures such as a balloon angioplasty or a stent deployment is captured in the filter 35. On completion of the procedure the filter (with any captured emboli) is retracted into an at least partially retracted configuration and withdrawn from the vasculature, for example using a retrieval catheter.

In the embodiments of the invention illustrated in Figs. 1 to 13 a rapid exchange configuration is used. In this case the guidewire may be clamped between the stabiliser 26 and a Touhy Borst 20. The position of the guidewire is therefore fixed relative to the Touhy Borst 20 and the guide catheter 30.

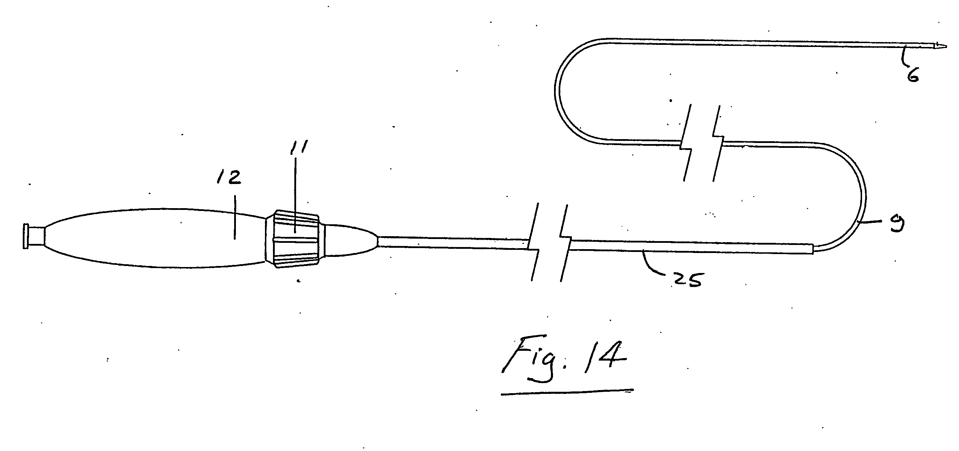

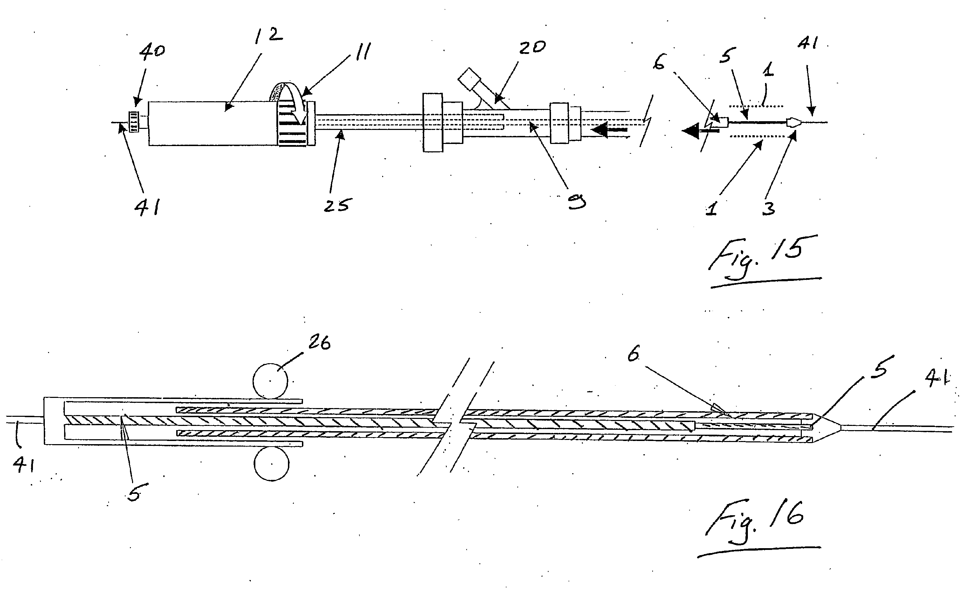

Referring to Figs. 14 to 16 the stabiliser 25 may also be used in association with an over the wire stent delivery system. In this case a locking mechanism 40 may be provided, for example at the proximal end of the handle 12 to lock the guidewire 41 position. The locking mechanism 40 may, for example, include a collet type arrangement.

The invention provides a stent delivery system that is of very low profile, excellent deliverability, and which can be used for very accurate placement of a self expanding stent in a tortuous arterial anatomy. It is suitable for deployment of a stent in a diseased vessel that may have a very significant reduction in lumen diameter. This reduction in lumen diameter may be as a result of atherosclerotic material on the vessel wall or it may be a hyperplasia response to injury. The stent may be deployed in vessels which have tortuous passageways leading thereto. Such vessels include a carotid artery, a superficial femoral artery or a renal artery.

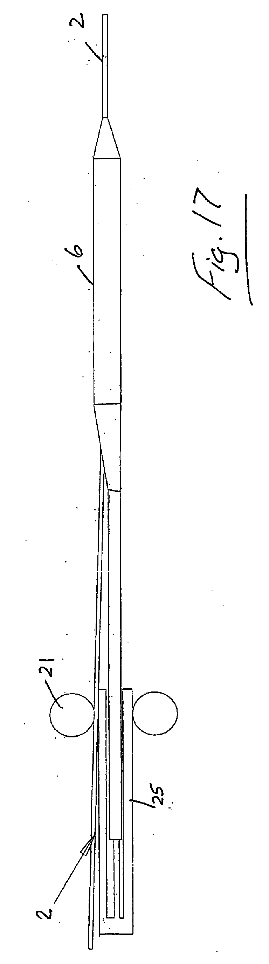

Another rapid exchange system is shown in Fig. 17.

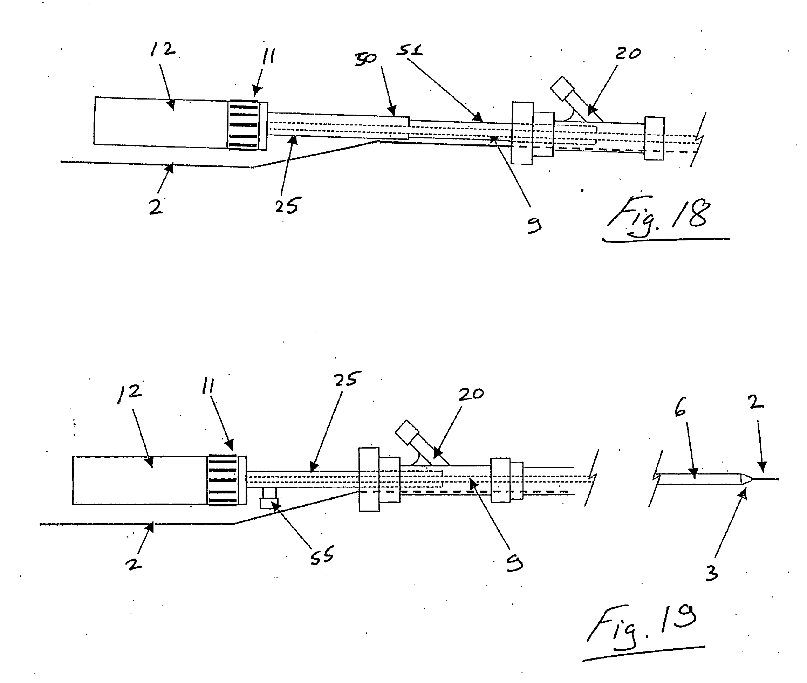

Referring now to Fig. 18 the stabiliser component 25 may comprise two or more telescoping parts 50, 51. This feature minimises the length of the stabiliser that needs to be inserted into the Touhy Borst 20 and prevents the stabiliser 25 from being inserted into the guide catheter 30.

Referring to Fig. 19 the stabiliser 25 in this case has a side port 55 distal of the handle 12. The side port 55 allows any backflow of blood from the guide catheter/Touhy Borst 20 to be vented prior to the handle 12. The side port 55 may have a standard female Luer adapter to provide additional means of injecting contrast media or other fluids into the guide catheter 30.

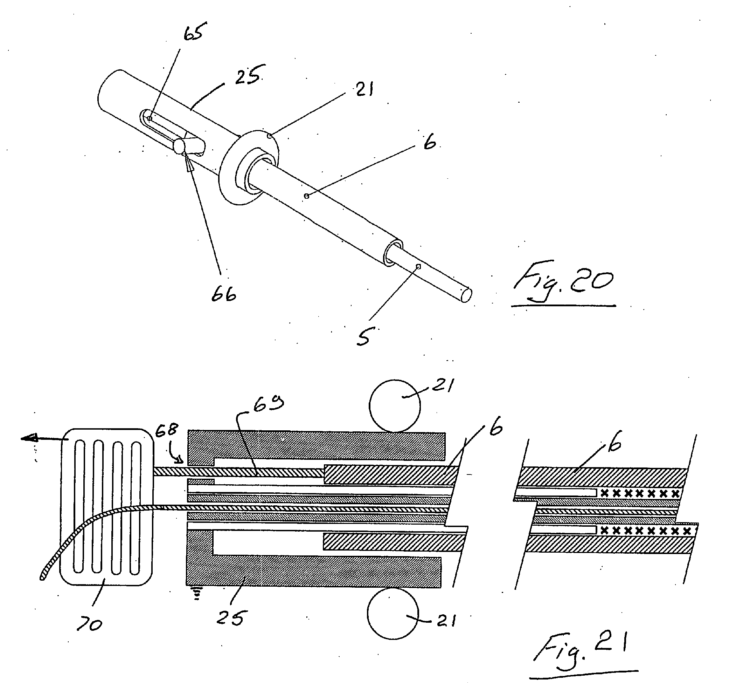

Fig. 20 shows a schematic of another embodiment of the invention. This figure shows the tubular housing 25 acting as both a stabilising tube and a user handle. The proximal section of tubular housing 25 remains outside the guide catheter or sheath while the distal end enters the sheath. The portion of tubular housing 25 proximal to the gasket 21 may be gripped by the user. A deployment pin 66 moves in slot 65 to deploy the stent. The gasket 21, of either the sheath or Touhy Borst (not shown), grips the outer surface of housing 25 and fixes the position of the stent during deployment. It will be appreciated that tubular housing 25 may extend much more distally of valve 21 than is shown in this schematic.

Deployment pin 66 is connected to catheter shaft 6. When the deployment pin 66 is moved proximally in the slot 65, the catheter shaft 6 slides proximally relative to the inner core 5. The inner core 5 engages the proximal end of the stent 1 and the stent 1 is restrained axially as the sheath is deployed.

In Fig. 21 there is illustrated an arrangement in which the stabiliser tube 25 has a proximal end port 68 through which a connector 69 extends. The connector 69 is connected to the outer sheath 6 at one end and has a proximal end operator actuating element 70. In use, the stabiliser tube 25 and/or the associated external mounting for the inner core 5 is held stationary while the operator actuating element is pulled back proximally from the mounting. The connector 69, which in this case extends through the proximal wall of the mounting and is in the form of a pull wire, connects the pull handle 75 to the outer sheath 6 of the catheter shaft and pulling on the handle 75 results in a corresponding retraction of the sheath 6 to expose the stent 1, on deployment. The action of the user corresponds to the action of the sheath and is therefore easy and intuitive for a clinician. The external mounting is stationary whilst the pull handle 75 is pulled proximally. This system may or may not include a suitable stabiliser component as described in any of the embodiments or drawings herein.

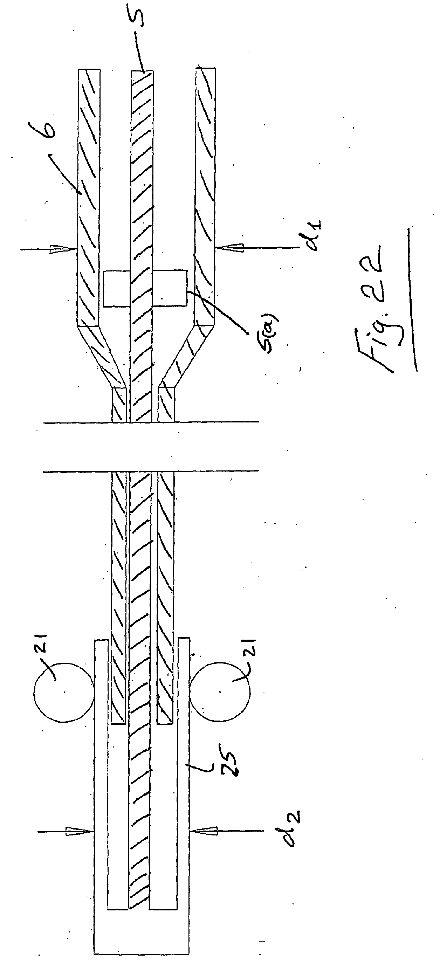

Referring to Fig. 22 a stabiliser 25 has an outside diameter d₂ that is less than the diameter d₁ of the distal end of the delivery system. Thus, the addition of the stabiliser 25 does not affect the profile of the system. This is an important feature in ensuring a low profile system and may be applied to all embodiments of the invention.

In Fig. 22 the abutment or step which is engaged against the proximal end of the stent 1 is illustrated as a simple lateral projection 5a on the inner core 5. This does not fall within the scope of the claims.

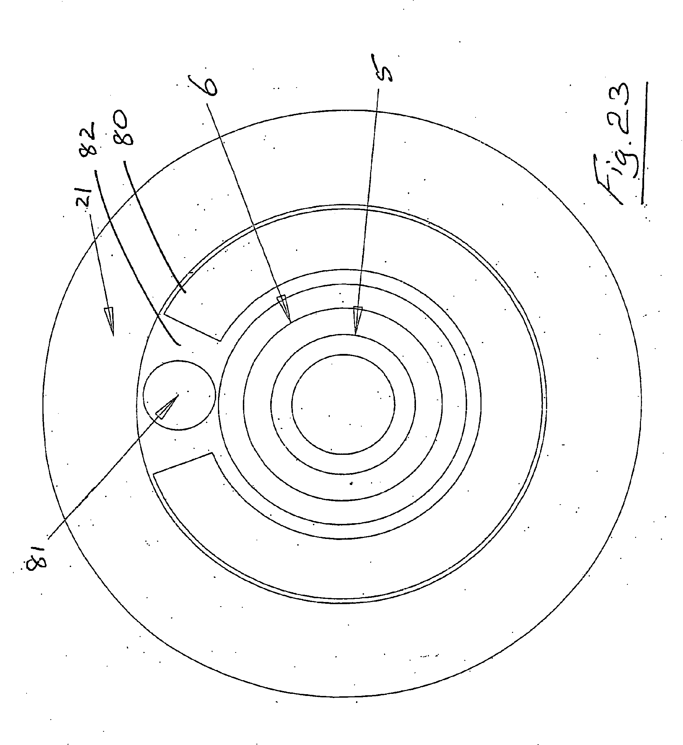

Fig. 23 illustrates an embodiment in which a stabiliser 80 has an elongate opening 82 to receive a guidewire 81. This arrangement is particularly suitable for rapid exchange. The wall thickness of the stabiliser 80 may be larger than the diameter of the guidewire 81 and the slot or gap 82 in the wall ensures that the guidewire is not clamped when the Touhy Borst 20 is tightened.

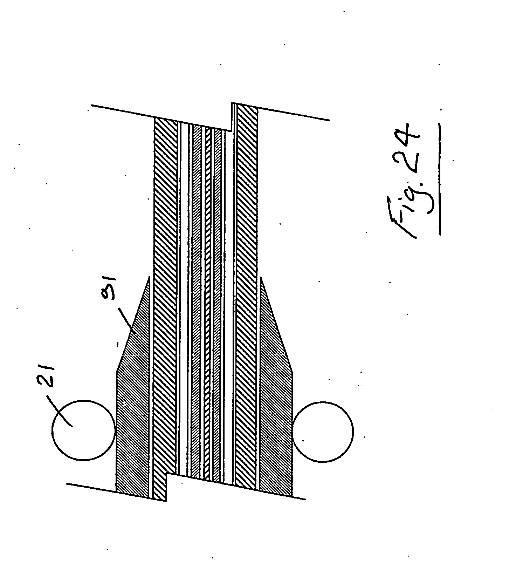

Referring to Fig. 24, a stabilising tube 90 may have a tapered distal end 91 to provide a smooth transition as the stabiliser 90 is inserted into a Touhy Borst 20.

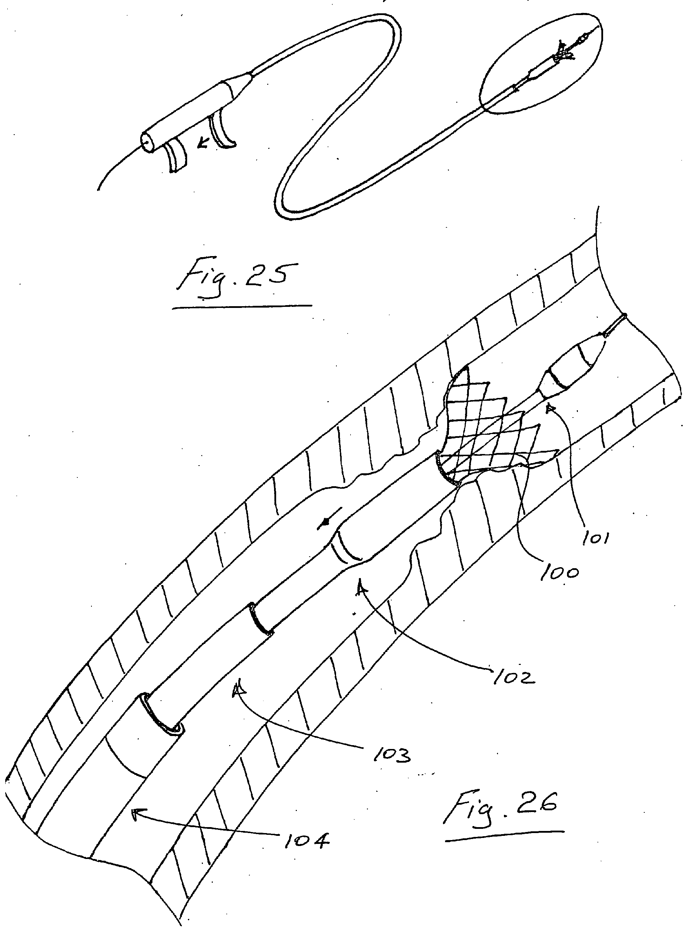

In Figs. 25 and 26 there is illustrated another stent delivery system not according to the invention for a stent 100. The delivery system comprises an inner core 101, a retractable middle sheath 102, a stent delivery outer 103 and a guide catheter 104. Conventional 2-layer systems do not make it easy for the user to hold the stent and inner core in a fixed position while retracting the outer layer to deploy the stent. Friction between the outer layer and the guide catheter and its fittings also hinder smooth deployment. In this embodiment of the invention a 3-layer delivery system is provided and the outer layer of the device which the user sees and holds remains in a fixed position relative to the guide catheter. The stent is deployed by operating a simple actuator (push/pull/trigger/rotate) 105 which retracts the middle sheath 102 relative to the outer layer 103 and inner core 101. In this case the stabiliser extends up to the sheath. This avoids the necessity for a lock at the proximal end. The stabiliser itself may have a distal region for a pod.

It will be noted that in this case the stabiliser component extends substantially the length of the catheter shaft. The stabiliser component can terminate a short distance (twice the length of the stent) from the distal end of the catheter shaft. The stabiliser is sufficiently long that it generates a lot of friction with the inner (such as a guide catheter and/or catheter shaft). Thus, the stabiliser component will stay substantially stationary, in use, during stent deployment.

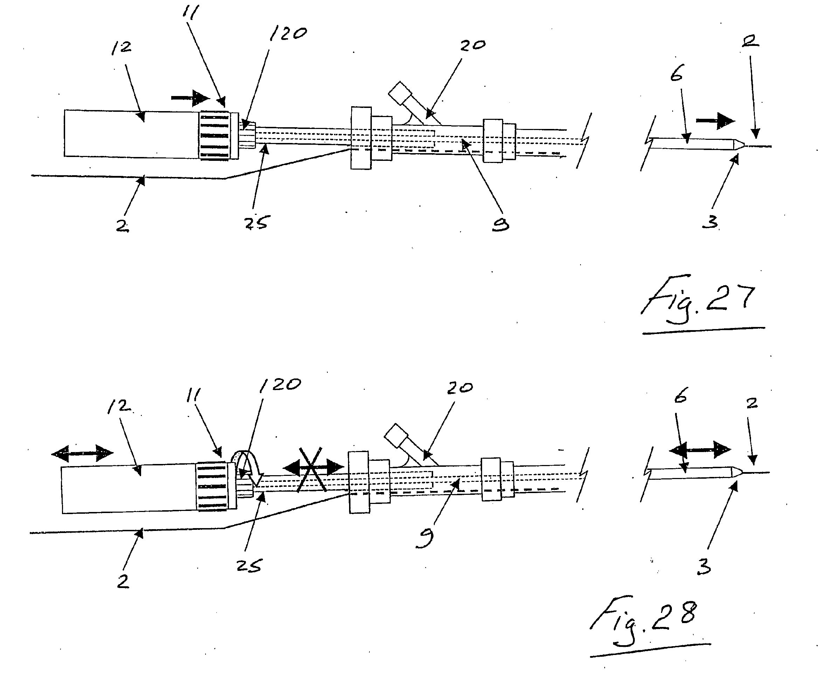

Referring to Figs. 27 and 28 there is illustrated another stent deployment system which is similar to that of Fig. 9 and like parts are assigned the same reference numerals. In this case the system has a position control device 120. The delivery catheter is advanced to the deployment site, causing the stabiliser 25 to enter the Touhy Borst 20 which is tightened to the stabiliser 25. The guidewire 2 is clamped between the stabiliser 25 and the Touhy Borst 20 thereby fixing the position of the tip 3 and the guidewire 2 relative to the Touhy Borst 20 and guide catheter. The position control device 120 allows readjustment of the catheter tip 3 before deployment without the necessity of opening the Tuohy Borst. The control device 120 may be used to move the handle (and hence the inner core assembly and stent position) proximally or distally relative to the guide catheter. The control device 120 may be implemented, for example, as a thumbscrew located on the same axis as that of the stabiliser 25. It may also be implemented as a push/pull mechanism. A user may use one hand on the handle with the free hand operating the position control device 120. The user may also open the Tuohy Borst and then readjust the position of the catheter, without necessarily using the position control device. The advantage of the position control device 120 is that it can be used to increase deployment accuracy as small adjustments may be made to the position of the inner core/outer core (and hence the position of the stent) prior to deployment and/or during deployment up to the stage at which the stent contacts a vessel wall without the necessity of opening the introducer valve.

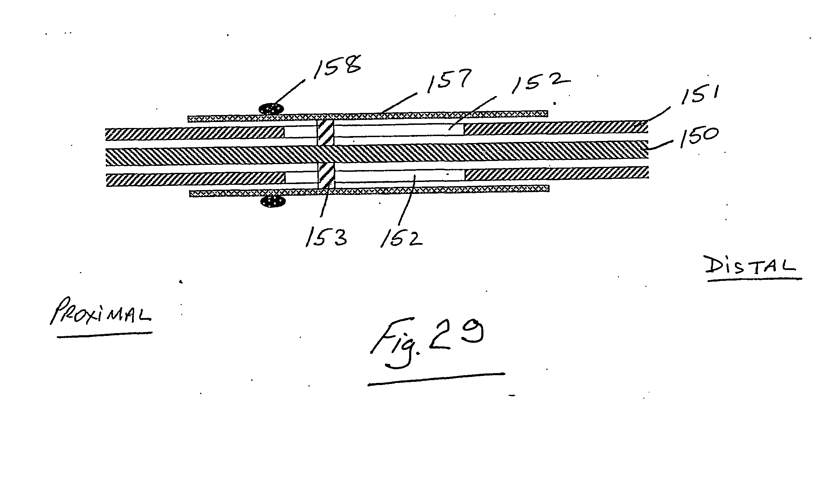

Fig. 29 is a cross sectional view of a stent deployment system with an inner member 150 such as a tube or a wire and a catheter shaft 151. The shaft 151 has through slots 152 through which a connector 153 extends between the inner member 150 and a stabiliser tube 157. The stabiliser tube 157 is engaged with a gasket 158. The connector 153 fixes the inner member 150 to the stabiliser tube 157 even though there is not necessarily any connection between the stabiliser tube and the handle (not shown). The slots 152 facilitate relative movement between the inner member 150 and the shaft 151 for stent deployment.

The stabiliser component used in the invention has an added advantage of creating an anti-backbleed feature. With the current set-up the Touhy Borst is required to be open so that the sheath can be retracted, this leads to the loss of blood through the Touhy Borst. This is prevented with the stabiliser as the Touhy Borst is tightened preventing the loss of blood. The blood is prevented from flowing down through the gap between the stabiliser and the catheter shaft because the gap between the tubes is very small and because the length of the stabiliser is long enough so that it resists the blood flow down the gap between the tubes.

The invention is not limited to the embodiments hereinbefore described which may by varied in detail.

## Claims

1. A delivery system for delivery and deployment of a self expanding stent (1) at a desired vascular location of a patient, the system comprising:
a self expanding stent (1), the stent having a proximal end and a distal end;
a catheter shaft (9) having a proximal end and a distal end, the distal end of the shaft defining a reception space for receiving the stent, the stent having a reduced diameter delivery configuration, the catheter shaft comprising a distal sheath portion (6) and a proximal shaft portion, the diameter of the proximal shaft portion being smaller than the diameter of the distal sheath portion and the distal sheath portion defining the reception space for the stent;
an inner core (5) having a proximal end and a distal end and a length, the stent being disposable about the distal end of the inner core;
an outer core (10) having a proximal end and a distal end and a length, the outer core being disposed radially about and affixed to the inner core, wherein the length of the outer core is less than the length of the inner core, the distal end of the outer core having an abutment which is engagable with the proximal end of the stent to deploy the stent;
an operator handle (12), for movement of the catheter shaft relative to the inner core and the outer core to deploy the self expanding stent;
a stabiliser component (25) comprising a tube having a distal end and a proximal end **characterised by**; the distal end of the stabiliser tube being disposed proximally to the stent;
the inner core and the outer core being fixed to the stabiliser tube;
the stabiliser tube being disposed over the smaller diameter proximal shaft and wherein the diameter of the stabiliser tube is not greater than the diameter of the distal sheath of the catheter shaft.

2. A delivery system as claimed in claim 1 wherein the inner core (5) has high compressive stiffness.

3. A delivery system as claimed in claim 2 wherein the inner core (5) is of a composite, or a metallic construction.

4. A delivery system as claimed in any of claims 1 to 3 wherein the catheter shaft (9) has a guidewire exit port which is located proximally or the distal end of the catheter shaft.

5. A delivery system as claimed in claim 4 wherein the guidewire exit port (7) is located proximally of the stent (1).

6. A delivery system as claimed in claim 4 or 5 wherein the guidewire exit port (7) is located proximally of the distale sheath (6).

7. A delivery system as claimed in any of claims 4 to 6 wherein the guidewire exit port (7) is located at a transition between the distal sheath and the reduced diameter proximal shaft portion.

8. A delivery system as claimed in any of claims 4 to 7 wherein the guidewire exit port (7) is located distally of the stabiliser tube (25).

9. A delivery system as claimed in any of claims 4 to 8 wherein the guidewire exit port (7) is configured to exit along an axis that is substantially parallel to a longitudinal axis of the distal sheath (6).

10. A delivery system as claimed in claim wherein the system comprises a guidewire (2) and the sum of the diameter of the guidewire and the diameter of the proximal shaft (9) is less than the diameter of the distal sheath (6)

11. A delivery system as claimed in claim 1 wherein the sum of the diameter of the guidewire (2) and the diameter of the stabiliser tube (25) is leass than the diameter of the distal sheath (6).

12. A delivery system as claimed in claim 1 wherein the inner core (5) comprises a large diameter distal segment, a reduced diameter proximal segment, and a transition segment between the distal and proximal segments, the transition segment may be proximal of the abutment region, the transition segment may be distal of the exit port.

13. A delivery system as claimed in claim 1 wherein the stent (1) direct)y engages the distal sheath (6) and is slidable relative to the sheath.

14. A delivery system as claimed in claim 13 wherein the distal sheath (6) is a composite with a low friction inner surface, the distal sheath may be reinforced to withstand the radial stresses of the stent in its constrained reduced diameter configuration.

15. A system as claimed in any of claims 1 to 14 wherein the inner core (5) is fixed to a component of the delivery system.

16. A system as claimed in any of claims 1 to 15 wherein the component of the system to which the inner core (5) is fixed comprises the handle (12).

17. A system as claimed in any of claims 1 to 16 wherein the stabiliser tube (25) is fixed to a procedural catheter.

18. A system as claimed in claim 17 wherein a haemostasis gasket is provided between the stabiliser tube and the procedural catheter, the catheter may be an introducer sheath which may have an integral haemostasis gasket (21).

19. A system as claimed in claim 18 wherein the procedural catheter is a guide catheter (30), the guide catheter may have a haemostasis gasket attachment, the gasket may be adjustable by the operator, the gasket attachment may be a Touchy Borst (20).

20. A system as claimed in any of claims 1 to 19 wherein the system comprises a procedural guidewire and the guidewire is fixed or fixable to the stabiliser tube (25).

21. A system as claimed in any of claims 1 to 20 wherein the stabiliser tube (25) is length adjustable.

22. A system as claimed in any of claims 1 to 21 wherein the stabiliser tube comprises at least two parts (50, 51) which are movable relative to one another.

23. A system as claimed in claim 1 wherein the stabiliser tube (25) position is adjustable.

24. A system as claimed in claim 23 wherein the srabiliser tube (25) is adjusted by rotation of a threaded element which provides a position control device.

25. A system as claimed in any of claims 1 ta 24 wherein an intermediate component is provided between the stabiliser tube (25) and the inner core (5).

26. A system as claimed in claim 25 wherein the intermediate component comprises the handle (12).

27. A system as claimed in claim 25 wherein the intermediate component comprises at least one bridging piece, the bridging piece may extend through the wall of the proximal shaft, the bridging piece may project laterally of the inner core and/or the stabiliser tube, the bridging piece may project radially between the inner core (5) and the stabiliser tube (25).

28. A system as claimed in claim 1 wherein the stabiliser tube (25) and the inner core (5) are directly mounted to one another.

29. A system as claimed in claim 28 wherein the stabiliser tube (25) is melded to the inner core, the stabiliser tube may be melded by a welding, gluing, joining, laminating, or bonding process.

30. A system as claimed in claim 28 or 29 wherein the stabiliser tube (25) and the inner core (5) are directly mounted to one another proximal of the distal outer sheath (6), the stabiliser tube and the inner core may be directly mounted to one another proximal of the outer shaft.

31. A system as claimed in claim 1 wherein the system includes a guidewire (2) and the guidewire extends at least the length of the catheter shaft (9).

32. A system as claimed in claim 31 wherein the inner core (5) defines a guidewire lumen (8a) along the length thereof.

33. A system as claimed in claim 31 or 32 wherein the system includes a lock (40) tor the guidewire, the lock may be located proximal of the handle (12).

34. A system as claimed in claim 1 wherein the stabiliser tube has a tapered distal end (91).

35. A system as claimed in claim 1 wherein the system includes a guidewire (3) and the guidewire is located within the profile of the stabiliser tube (25).

36. A system as claimed in claim 1 wherein the stabliser tube (25) has a proximal opening to allow backflow of blood.

## Patentansprüche

1. Verabreichungssystem zum Verabreichen und Entfalten eines selbstexpandierenden Stents (1) an einer gewünschten Gefäßstelle eines Patienten, wobei das System Folgendes umfasst:
einen selbstexpandierenden Stent (1) wobei der Stent ein proximales Ende und ein distales Ende aufweist;
einen Katheterschaft (9) mit einem proximalen Ende und einem distalen Ende, wobei das distale Ende des Schafts einen Aufnahmeraum zum Aufnehmen des Stents definiert, wobei der Stent eine Verabreichungskonfiguration mit verringertem Durchmesser aufweist, wobei der Katheterschaft einen distalen Hüllabschnitt (6) und einen proximalen Schaftabschnitt aufweist, wobei der Durchmesser des proximalen Schaftabschnitts kleiner ist als der Durchmesser des distalen Hüllabschnitts und der distale Hüllabschnitt den Aufnahmeraum für den Stent definiert;
einen inneren Kern (5) mit einem proximalen Ende und einem distale Ende und einer Länge, wobei der Stent um das distale Ende des inneren Kerns angeordnet werden kann;
einen äußeren Kern (10) mit einem proximalen Ende und einem distalen Ende und einer Länge, wobei der äußere Kern radial um den inneren Kern angeordnet ist und daran befestigt ist, wobei die Länge des äußeren Kerns geringer ist als die Länge des inneren Kerns, wobei das distale Ende des äußeren Kerns eine Raste aufweist, die mit dem proximalen Ende des Stents in Eingriff treten kann, um den Stent zu entfalten;
einen Bedienergriff (12) zum Bewegen des Katheterschafts relativ zu dem inneren Kern und dem äußeren Kern, um den selbstexpandierenden Stent zu entfalten;
eine Stabilisatorkomponente (25), umfassend eine Röhre mit einem distalen Ende und einem proximalen Ende; **dadurch gekennzeichnet, dass** das distale Ende der Stabilisatorröhre proximal zu dem Stent angeordnet ist;
der inneren Kern und der äußere Kern an der Stabilisatorröhre fixiert sind;
die Stabilisatorröhre über dem proximalen Schaft mit kleinerem Durchmesser angeordnet ist und wobei der Durchmesser der Stabilisatorröhre nicht größer ist als der Durchmesser der distalen Hülle des Katheterschafts.

2. Verabreichungssystem nach Anspruch 1, wobei der innere Kern (5) eine hohe Drucksteifigkeit aufweist.

3. Verabreichungssystem nach Anspruch 2, wobei der innere Kern (5) einen Aufbau aus Verbundmaterial oder Metall aufweist.

4. Verabreichungssystem nach einem der Ansprüche 1 bis 3, wobei der Katheterschaft (9) eine Führungsdraht-Austrittsöffnung aufweist, die proximal zum distalen Ende des Katheterschafts liegt.

5. Verabreichungssystem nach Anspruch 4, wobei die Führungsdraht-Austrittsöffnung (7) proximal zu dem Stent (1) liegt.

6. Verabreichungssystem nach Anspruch 4 oder 5, wobei die Führungsdraht-Austrittsöffnung (7) proximal zu der distalen Hülle (6) liegt.

7. Verabreichungssystem nach einem der Ansprüche 4 bis 6, wobei die Führungsdraht-Austrittsöffnung (7) an einem Übergang zwischen der distalen Hülle und dem proximalen Schaftabschnitt mit verringertem Durchmesser liegt.

8. Verabreichungssystem nach einem der Ansprüche 4 bis 7, wobei die Führungsdraht-Austrittsöffnung (7) distal von der Stabilisatorröhre (25) liegt.

9. Verabreichungssystem nach einem der Ansprüche 4 bis 8, wobei die Führungsdraht-Austrittsöffnung (7) dazu konfiguriert ist, entlang einer Achse auszutreten, die im Wesentlichen zu einer Längsachse der distalen Hülle (6) parallel ist.

10. Verabreichungssystem nach Anspruch 1, wobei das System einen Führungsdraht (2) umfasst und die Summe des Durchmessers des Führungsdrahts und des Durchmessers des proximalen Schafts (9) geringer ist als der Durchmesser der distalen Hülle (6).

11. Verabreichungssystem nach Anspruch 1, wobei die Summe des Durchmessers des Führungsdrahts (2) und des Durchmessers der Stabilisatorröhre (25) geringer ist als der Durchmesser der distalen Hülle (6).

12. Verabreichungssystem nach Anspruch 1, wobei der innere Kern (5) ein distales Segment mit großem Durchmesser, ein proximales Segment mit verringertem Durchmesser und ein Übergangssegment zwischen dem distalen und dem proximalen Segment umfasst, wobei das Übergangs segment proximal zu der Rastregion sein kann und das Übergangssegment distal von der Austrittsöffnung sein kann.

13. Verabreichungssystem nach Anspruch 1, wobei der Stent (1) direkt mit der distalen Hülle (6) in Eingriff steht und relativ zu der Hülle gleiten kann.

14. Verabreichungssystem nach Anspruch 13, wobei es sich bei der distalen Hülle (6) um ein Verbundmaterial mit einer reibungsarmen inneren Oberfläche handelt, wobei die distale Hülle verstärkt sein kann, um den radialen Beanspruchungen des Stents in seiner eingezwängten Konfiguration mit verringertem Durchmesser zu widerstehen.

15. System nach einem der Ansprüche 1 bis 14, wobei der innere Keren (5) an einer Komponente des Verabreichungssystems fixiert ist.

16. System nach einem der Ansprüche 1 bis 15, wobei die Komponente des Systems, an der der innere Kern (5) fixiert ist, den Griff (12) umfasst.

17. System nach einem der Ansprüche 1 bis 16, wobei die Stabilisatorröhre (25) an einem Operationskatheter fixiert ist.

18. System nach Anspruch 17, wobei eine Hämostasedichtung zwischen der Stabilisatorröhre und dem Operationskatheter bereitgestellt ist und es sich bei dem Katheter um eine Einführungshülle handeln kann, die eine integrierte Hämostasedichtung (21) aufweisen kann.

19. System nach Anspruch 18, wobei es sich bei dem Operationskatheter um einen Führungskatheter (30) handelt, der Führungskatheter ein Hämostasedichtungs-Anbauteil aufweisen kann, die Dichtung von der Bedienperson verstellbar sein kann und es sich bei dem Dichtungsanbauteil um einen Tuohy-Borst (20) handeln kann.

20. System nach einem der Ansprüche 1 bis 19, wobei das System einen Operationsführungsdraht umfasst und der Führungsdraht an der Stabilisatorröhre (25) fixiert ist oder fixiert werden kann.

21. System nach einem der Ansprüche 1 bis 20, wobei die Stabilisatorröhre (25) längenverstellbar ist.

22. System nach einem der Ansprüche 1 bis 21, wobei die Stabilisatorröhre mindestens zwei Teile (50, 51) umfasst, die relativ zueinander beweglich sind.

23. System nach Anspruch 1, wobei die Lage der Stabilisatorröhre (25) verstellbar ist.

24. System nach Anspruch 23, wobei die Stabilisatorröhre (25) durch Drehung eines Gewindeelements verstellt wird, das eine Lagesteuerungsvorrichtung bereitstellt.

25. System nach einem der Ansprüche 1 bis 24, wobei eine Zwischenkomponente zwischen der Stabilisatorröhre (25) und dem inneren Kern (5) bereitgestellt ist.

26. System nach Anspruch 25, wobei die Zwischenkomponente den Griff (12) umfasst.

27. System nach Anspruch 25, wobei die Zwischenkomponente mindestens ein Überbrückungsstück umfasst, wobei sich das Überbrückungsstück durch die Wand des proximalen Schafts erstrecken kann, das Überbrückungsstück seitlich von dem inneren Kern und/oder der Stabilisatorröhre vorstehen kann, und wobei das Überbrückungsstück radial zwischen dem inneren Kern (5) und der Stabilisatorröhre (25) vorstehen kann.

28. System nach Anspruch 1, wobei die Stabilisatorröhre (25) und der innere Kern (5) direkt aneinander angebracht sind.

29. System nach Anspruch 28, wobei die Stabilisatorröhre (25) an den inneren Kern angefügt ist, wobei die Stabilisatorröhre durch einen Schweiß-, Klebe-, Verbindungs-, Laminier- oder Bondingprozess gefügt sein kann.

30. System nach Anspruch 28 oder 29, wobei die Stabilisatorröhre (25) und der innere Kern (5) proximal zu der distalen äußeren Hülle (6) direkt aneinander angebracht sind und die Stabilisatorröhre und der innere Kern proximal zu dem äußeren Schaft direkt aneinander angebracht sein können.

31. System nach Anspruch 1, wobei das System einen Führungsdraht (2) umfasst und sich der Führungsdraht mindestens über die Länge des Katheterschafts (9) erstreckt.

32. System nach Anspruch 31, wobei der innere Kern (5) ein Führungsdrahtlumen (8a) entlang der Länge desselben definiert.

33. System nach Anspruch 31 oder 32, wobei das System eine Arretierung (40) für den Führungsdraht umfasst, wobei die Arretierung proximal zu dem Griff (12) liegen kann.

34. System nach Anspruch 1, wobei die Stabilisatorröhre ein konisch zulaufendes distales Ende (91) aufweist.

35. System nach Anspruch 1, wobei das System einen Führungsdraht (2) umfasst und der Führungsdraht innerhalb des Profils der Stabilisatorröhre (25) liegt.

36. System nach Anspruch 1, wobei die Stabilisatorröhre (25) eine proximale Öffnung aufweist, um den Rückfluss von Blut zuzulassen.

## Revendications

1. Système de pose destiné à la pose et au déploiement d'un stent auto-expansible (1) à un emplacement vasculaire souhaité d'un patient, le système comprenant :
un stent auto-expansible (1), le stent ayant une extrémité proximale et une extrémité distale ;
un axe de cathéter (9) ayant une extrémité proximale et une extrémité distale, l'extrémité distale de l'axe définissant un espace de réception destiné à recevoir le stent, le stent ayant une configuration de pose de diamètre réduit, l'axe de cathéter comprenant une partie de gaine distale (6) et une partie d'axe proximale, le diamètre de la partie d'axe proximale étant inférieur au diamètre de la partie de gaine distale et la partie de gaine distale définissant l'espace de réception du stent ;
une âme interne (5) ayant une extrémité proximale et une extrémité distale ainsi qu'une longueur, le stent pouvant être disposé autour de l'extrémité distale de l'âme interne ;
une âme externe (10) ayant une extrémité proximale et une extrémité distale ainsi qu'une longueur, l'âme externe étant disposée radialement autour de l'âme interne et fixée à celle-ci, la longueur de l'âme externe étant inférieure à la longueur de l'âme interne, l'extrémité distale de l'âme externe ayant une butée qui peut être mise en prise avec l'extrémité proximale du stent pour déployer le stent ;
une poignée d'opérateur (12), destinée au mouvement de l'axe de cathéter par rapport à l'âme interne et à l'âme externe pour déployer le stent auto-gonflant ;
un composant stabilisateur (25) comprenant un tube ayant une extrémité distale et une extrémité proximale ; **caractérisé en ce que** l'extrémité distale du tube stabilisateur est disposée à une position proximale par rapport au stent ;
l'âme interne et l'âme externe sont fixées au tube stabilisateur ;
le tube stabilisateur est disposé par-dessus l'axe proximal de diamètre inférieur et dans lequel le diamètre du tube stabilisateur n'est pas supérieur au diamètre de la gaine distale de l'axe de cathéter.

2. Système de pose selon la revendication 1, dans lequel l'âme interne (5) a une haute rigidité en compression.

3. Système de pose selon la revendication 2, dans lequel l'âme interne (5) est un composite ou une construction métallique.

4. Système de pose selon l'une quelconque des revendications 1 à 3, dans lequel l'axe de cathéter (9) a un orifice de sortie de fil de guidage situé à une position proximale par rapport à l'extrémité distale de l'axe de cathéter.

5. Système de pose selon la revendication 4, dans lequel l'orifice de sortie de fil de guidage (7) est situé à une position proximale par rapport au stent (1).

6. Système de pose selon la revendication 4 ou 5, dans lequel l'orifice de sortie de fil de guidage (7) est situé à une position proximale par rapport à la gaine distale (6).

7. Système de pose selon l'une quelconque des revendications 4 à 6, dans lequel l'orifice de sortie de fil de guidage (7) est situé au niveau d'une transition entre la gaine distale et la partie d'axe proximale de diamètre réduit.

8. Système de pose selon l'une quelconque des revendications 4 à 7, dans lequel l'orifice de sortie de fil de guidage (7) est situé à une position distale par rapport au tube stabilisateur (25).

9. Système de pose selon l'une quelconque des revendications 4 à 8, dans lequel l'orifice de sortie de fil de guidage (7) est configuré pour sortir le long d'un axe qui est sensiblement parallèle à un axe longitudinal de la gaine distale (6).

10. Système de pose selon la revendication 1, le système comprenant un fil de guidage (2) et la somme du diamètre du fil de guidage et du diamètre de l'axe proximal (9) étant inférieur au diamètre de la gaine distale (6).

11. Système de pose selon la revendication 1, dans lequel la somme du diamètre du fil de guidage (2) et du diamètre du tube stabilisateur (25) est inférieure au diamètre de la gaine distale (6).

12. Système de pose selon la revendication 1, dans lequel l'âme interne (5) comprend un segment distal de grand diamètre, un segment proximal de diamètre réduit, et un segment de transition entre les segments distal et proximal, le segment de transition peut être proximal par rapport à la région de butée, le segment de transition peut être distal par rapport à l'orifice de sortie.

13. Système de pose selon la revendication 1, dans lequel le stent (1) s'enclenche directement avec la gaine distale (6) et peut coulisser par rapport à la gaine.

14. Système de pose selon la revendication 13, dans lequel la gaine distale (6) est un composite à surface interne de faible friction, la gaine distale peut être renforcée pour résister aux contraintes radiales du stent dans sa configuration de diamètre réduit contrainte.

15. Système selon l'une quelconque des revendications 1 à 14, dans lequel l'âme interne (5) est fixée à un composant du système de pose.

16. Système selon l'une quelconque des revendications 1 à 15, dans lequel le composant du système auquel l'âme interne (5) est fixée comprend la poignée (12).

17. Système selon l'une quelconque des revendications 1 à 16, dans lequel le tube stabilisateur (25) est fixé à un cathéter d'intervention.

18. Système selon la revendication 17, dans lequel une joint hémostatique est fourni entre le tube stabilisateur et le cathéter d'intervention, le cathéter pouvant être une gaine d'introduction pouvant être un joint hémostatique intégré (21).

19. Système selon la revendication 18, dans lequel le cathéter d'intervention est un cathéter de guidage (30), le cathéter de guidage peut comporter une fixation de joint hémostatique, le joint peut être réglé par l'opérateur, la fixation de joint pouvant être du type Tuohy Borst (20).

20. Système selon l'une quelconque des revendications 1 à 19, le système comprenant un fil de guidage d'intervention et le fil de guidage étant fixé ou pouvant être fixé au tube stabilisateur (25).

21. Système selon l'une quelconque des revendications 1 à 20, dans lequel le tube stabilisateur (25) est réglable en longueur.

22. Système selon l'une quelconque des revendications 1 à 21, dans lequel le tube stabilisateur comprend au moins deux parties (50, 51) qui sont mobiles l'une par rapport à l'autre.

23. Système selon la revendication 1, dans lequel la position du tube stabilisateur (25) est réglable.

24. Système selon la revendication 23, dans lequel le tube stabilisateur (25) est réglé par la rotation d'un élément fileté qui constitue un dispositif de contrôle de position.

25. Système selon l'une quelconque des revendications 1 à 24, dans lequel un composant intermédiaire est fourni entre le tube stabilisateur (25) et l'âme interne (5).

26. Système selon la revendication 25, dans lequel le composant intermédiaire comprend la poignée (12).

27. Système selon la revendication 25, dans lequel le composant intermédiaire comprend au moins une pièce de pontage, ladite pièce de pontage pouvant s'étendre à travers la paroi de l'axe proximal, la pièce de pontage pouvant saillir latéralement de l'âme interne et/ou du tube stabilisateur, la pièce de pontage pouvant saillir radialement entre l'âme interne (5) et le tube stabilisateur (25).

28. Système selon la revendication 1, dans lequel le tube stabilisateur (25) et l'âme interne (5) son montés directement l'un sur l'autre.

29. Système selon la revendication 28, dans lequel le tube stabilisateur (25) est fusionné avec l'âme interne, le tube stabilisateur pouvant être fusionné par un processus de soudage, collage, jointure, stratification, ou liaison.

30. Système selon la revendication 28 ou 29, dans lequel le tube stabilisateur (25) et l'âme interne (5) sont montés directement l'un sur l'autre à une position proximale par rapport à la gaine externe distale (6), le tube stabilisateur et l'âme interne pouvant être montés directement l'un sur l'autre à une position proximale par rapport à l'axe externe.

31. Système selon la revendication 1, dans lequel le système comporte un fil de guidage (2) et le fil de guidage s'étend sur au moins la longueur de l'axe de cathéter (9).

32. Système selon la revendication 31, dans lequel l'âme interne (5) définit une lumière de fil de guidage (8a) le long de sa longueur.

33. Système selon la revendication 31 ou 32, le système comportant un verrou (40) pour le fil de guidage, le verrou pouvant être placé à une position proximale par rapport à la poignée (12).

34. Système selon la revendication 1, dans lequel le tube stabilisateur comporte une extrémité distale conique (91).

35. Système selon la revendication 1, le système comportant un fil de guidage (2) et le fil de guidage étant placé dans le profil du tube stabilisateur (25).

36. Système selon la revendication 1, dans lequel le tube stabilisateur (25) comporte une ouverture proximale pour permettre le reflux du sang.
